# EUROPEAN PATENT APPLICATION

(11) **EP 0 654 269 A1**
(43) Date of publication of application: **24.05.1995**
(21) Application number: 94906768.0
(22) Date of filing: 05.08.1993
(51) Int. Cl.: A61K 31/70, C07H 19/10

(54) **MEDICINAL COMPOSITION FOR INHIBITING CANCER METASTASIS TO LIVER AND MEDICINAL COMPOSITION FOR CURING HEPATOMA**

(30) Priority: 10.08.1992 JP 232699/92; 18.06.1993 JP 170850/93
(71) Applicant: NIPPON KAYAKU KABUSHIKI KAISHA, Tokyo 102 (JP)
(72) Inventor: SATOH, Akira 1039, Kamiochiai, Saitama 338 (JP); EKIMOTO, Hisao 2-11-1-803, Shimo, Tokyo 115 (JP); OKAMOTO, Kazuya 5-7-10-305, Higashiogu, Tokyo 116 (JP); KOBAYASHI, Fumiko 3-13-9-602, Furuishiba, Tokyo 135 (JP); KUSANO, Syuichi 373-68, Oaza Fujikubo, Saitama 354 (JP); SASAKI, Hiroe Sherumu-Ai 202, Kita-ku Tokyo 114 (JP)
(74) Representative: Türk, Dietmar, Dr. rer. nat.
(86) International application number: JP9301100
(87) International publication number: WO9403183

(57) **Abstract**

Cytarabine ocphosphate represented by formula (I), which is effective in inhibiting the metastasis of cancer cells to the liver and hence is prospective as a cancer remedy.

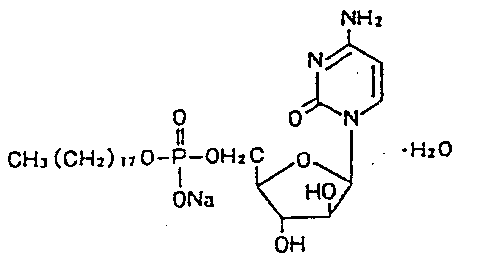

## Description

### TECHNICAL FIELD

The present invention relates to a medical use of Cytarabine Ocfosfate. More particularly, the present invention relates to a use of Cytarabine Ocfosfate for preventing liver metastasis of cancer and for the treatment of liver cancer.

### BACKGROUND ART

Chemotherapy has failed so far to provide any effective treatment for preventing liver metastasis of cancer accompanied by progress of cancer or surgical operation in patients with cancer. It is the actual situation that chemotherapy is relied upon supplementary treatment by anti-tumor agents, e.g., 5-FU or epiadriamycin. However, the effect of these anti-tumor agents is not satisfactory.

Cytarabine Ocfosfate (1-β-D-arabinofuranosylcytosine-5'-stearylphosphate monosodium salt monohydrate) is a derivative of Ara-C and known as an oral anti-tumor agent (Japanese Patent Application KOKOKU No. 57-48091 and U.S. Patent No. 4,542,021). It is also known that this compound is metabolized in liver to Ara-C (Nagahata et al., Anti-tumor Effect of Novel Cytarabine derivative 4-Amino-1-β-D-Arabinofuranosyl-2(1H)-Pyrimidinone 5'-(Sodium-Octadecyl Phosphate) Monohydrate (YNKO1), GAN-TO-KAGAKU-RYOHO (Cancer & Chemotherapy), 17 (8), 1437-1445 (1990); Nakagawa Y. et al., Studies on the metabolism of YNKO1 (in Japanese), in Proceedings of 106th annual meeting of the Japan Pharmaceutical Society, p. 528 (1986)).

For radical treatment of cancer, it is important not only to treat a primary site of the cancer but also to prevent metastasis of, especially gastrointestinal cancer or lung cancer to liver. It is thus desired a drug effective for preventing metastasis to liver. In addition, liver tumor including both metastatic and primary tumor is treated only with difficulty. It is also desired a drug effective for liver tumor, which can be continuously administered orally over a long period of time.

### DISCLOSURE OF INVENTION

The present inventors have found that Cytarabine Ocfosfate is excellent in oral absorption, has an activity for preventing liver metastasis of cancer, and is effective for liver cancer including both primary and metastatic cancer.

Therefore, an object of the present invention is to provide a pharmaceutical composition for preventing liver metastasis of cancer and a pharmaceutical composition for the treatment of liver cancer, comprising as an effective ingredient Cytarabine Ocfosfate represented by formula (1) below: 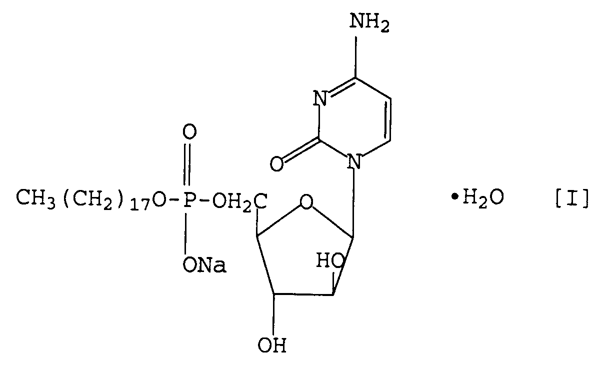
Another object of the present invention is to provide a method for preventing liver metastasis of cancer and a method for the treatment of liver cancer, which comprises administering an effective dose of Cytarabine Ocfosfate represented by formula (1).

A further object of the present invention is to provide use of Cytarabine Ocfosfate for preparing the pharmaceutical composition for preventing liver metastasis of cancer and the pharmaceutical composition for the treatment of liver cancer, comprising as an effective ingredient Cytarabine Ocfosfate represented by formula (1).

A still further object of the present invention is to provide a process for producing the pharmaceutical composition for preventing liver metastasis of cancer and the pharmaceutical composition for the treatment of liver cancer, which comprises mixing Cytarabine Ocfosfate as an effective ingredient with an excipient or a carrier.

### BEST MODE FOR CARRYING OUT THE INVENTION

Cytarabine Ocfosfate which is employed as an effective ingredient in the present invention can be produced by known processes (Japanese Patent Application KOKOKU Nos. 63-35639 and 63-30315, U.S. Patent No. 4,812,560).

Where Cytarabine Ocfosfate is used for preventing liver metastasis of cancer and for the treatment of liver cancer, the effective ingredient is administered alone or as admixture with an excipient or a carrier, in the form of an oral drug or a suppository and the like. As the excipient, pharmaceutically acceptable excipient is chosen; its kind and composition are determined depending upon route or method for application. Examples of the excipient which can be used are liquid carriers such as water, alcohols, or animal and vegetable oils like soybean oil, peanut oil, sesame oil, mineral oil, etc., or synthetic oil. Examples of solid carriers include sugars such as maltose, sucrose, etc.; amino acids; hydroxypropyl cellulose derivatives; organic acid salts such as magnesium stearate. The content of Cytarabine Ocfosfate in the pharmaceutical composition varies depending upon the form of preparation but is generally in the range of 0.01 to 100 wt%, preferably in the range of 0.1 to 50 wt%. In the case of oral administration, Cytarabine Ocfosfate is administered together with the solid carrier or liquid carrier described above, in the form of tablets, capsules, powders, granules, liquid, dry syrup, etc. Capsules, tablets, granules and powders contain the effective ingredient generally in the range of 5 to 100 wt%, preferably 25 to 98 wt%.

A preferred embodiment of the pharmaceutical composition for carrying out the present invention is in the form of Cytarabine Ocfosfate hard capsules comprising (1) Cytarabine Ocfosfate, (2) a high molecular weight compound which acts as a disintegrating agent, and (3) an alkali.

Any high molecular weight compound can be used as a disintegrating agent so long as it is pharmaceutically acceptable. A preferred compound is low substitution hydroxypropyl cellulose. The low substitution hydroxypropyl cellulose is used to mean cellulose having a low substitution degree of hydroxypropylation, e.g., the derivative described in Japanese Pharmacopeia, XIth revision, in which a rate of hydroxypropylation ranges from 7 to 16%.

The high molecular weight compounds which act as disintegrators are incorporated generally in the amount of approximately 0.5 to 4 parts by weight, preferably 1 to 3.5 parts by weight, more preferably 1.3 to 3.0 parts by weight, based on 1 part by weight of Cytarabine Ocfosfate.

The alkali is not particularly limited but usable so long as it can be employed as an additive for pharmaceutical use. Specific examples of the alkali include sodium carbonate, potassium carbonate, sodium hydrogencarbonate, sodium hydroxide, potassium hydroxide, etc. These alkalis may be used alone or as admixture of two or more. In general, sodium carbonate or potassium carbonate is preferably used. The alkali is incorporated in the amount of approximately 0.002 to 0.3 part by weight, preferably 0.005 to 0.2 part by weight, more preferably 0.007 to 0.07 part by weight, based on 1 part by weight of Cytarabine Ocfosfate.

Proportions of the respective ingredients packed in a hard capsule are 5 to 50 W/W % of Cytarabine Ocfosfate, preferably 10 to 47 W/W %, more preferably 20 to 40 W/W %; 10 to 80 W/W % of the high molecular weight compound acting as a disintegrator, preferably 15 to 75 W/W %, more preferably 30 to 70 W/W %; and 0.1 to 8.0 W/W % of the alkali, preferably 0.2 to 4.0 W/W %. The balance is other additives which are incorporated in the amount of 0 to 84 W/W %; preferably 1 to 74 W/W %.

In order to obtain hard capsule preparations, other additives, e.g., an excipient, a binder and a lubricant, may be appropriately incorporated in the composition. Examples of the excipient include starches such as corn starch, potato starch, wheat, etc., sugars such as lactose, mannitol, glucose, etc. The excipient may be incorporated in the amount of approximately 0.5 to 7 parts by weight, preferably 1 to 5 parts by weight, based on 1 part by weight of Cytarabine Ocfosfate.

Examples of the binder include water-soluble cellulose ether derivatives, e.g., hydroxypropyl cellulose, methyl cellulose, etc.; and polyvinylpyrrolidone, sodium alginate, starch glue, aminoalkyl methacrylate copolymer (Endragit), gum arabic, etc. The binder may be incorporated in the amount of approximately 0.005 to 0.2 part by weight, preferably 0.01 to 0.1 part by weight, based on 1 part by weight of Cytarabine Ocfosfate. Furthermore, as the lubricant there may be employed stearic acid and stearates such as magnesium stearate, etc., talc, leucine, carnauba wax, cacao fat, polyethylene glycol, cetanol, paraffin, etc. The lubricant may be incorporated in the amount of approximately 0.005 to 0.10 part by weight, preferably 0.009 to 0.05 part by weight, based on 1 part by weight of Cytarabine Ocfosfate.

For producing hard capsule preparations, it is preferred to use as Cytarabine Ocfosfate stable crystals of Cytarabine Ocfosfate having no hygroscopicity as disclosed in Japanese Patent Application KOKOKU No. 63-30315 and U.S. Patent No. 4,812,560. The respective components are filled up in hard capsules as follows. The components are generally granulated by wet granulation or dry granulation and the resulting granules are packed in hard capsules to obtain the objective hard capsules.

A dose is determined depending upon age, body weight, conditions of the patient and purpose of treatment, etc. A therapeutic dose is generally in the range of 25 to 500 mg/day/adult, preferably 50 to 300 mg/day/adult, for oral administration. Especially in the case of preventing metastasis, the composition is preferably administered orally in a small dose (e.g., 50 to 100 mg/day/adult) over a long period of time.

Next, the pharmacological properties of Cytarabine Ocfosfate which is used as the effective ingredient in the present invention are demonstrated with reference to the following experiment example. (Experiment Example)

### Effect of preventing metastasis to mouse colony tumor Colon 26

### Method:

Tumor cells are transplanted in a mouse spleen to form metastatic site selectively in the liver. It is reported that various experiments for treatment can be made for the purpose of controlling liver metastasis (Kiyoshi MORIKAWA, "Transplantation of human cancer in the spleen of nude mouse as a model for liver metastasis", Oncologia, 22(2), 100-102 (1989); L. Kopper et al., Experimental Model for Liver Metastasis Formation Using Lewis Lung Tumor, Journal of Cancer Research Clinical Oncology, 103: 31-38 (1982)).

Using the method, the present inventors prepared an experimental model for liver metastasis of mouse colony tumor Colon 26 and evaluated the activity of Cytarabine Ocfosfate for preventing liver metastasis in comparison with Yamasa Cytarabine (Yamasa Shoyu K.K., ara-C), in terms of a rate of surviving days of the treated group to surviving days of the control group (prolonged life span: T/C %).

The drug was given one day after transplantation of tumor cells. The effective ingredient was orally administered for consecutive five (5) days in a dose of 160 mg/kg/day calculated as Cytarabine Ocfosfate, and orally in a dose of 200 mg/kg/day calculated as ara-C for consecutive five (5) days.

As shown in Table 1, Cytarabine Ocfosfate showed a remarkable effect prolonging life span, as compared to ara-C, indicating the metastasis prevention effect. It was confirmed by autopsy of dead mice that the liver was suffered from tumor in all cases so that the animal was bled to death and no metastasis to other organs was noted.

**Table 1**

| Prevention effect on liver metastasis of mouse colony tumor Colon 26 | | | |
|---|---|---|---|
| Group | Dose | Median Number of Surving Days | T/C (%) |
| Control group | | 14.0 | 100 |
| Cytarabine Ocfosfate | 160 mg/kg/day | 29.0 | 207 |
| ara-C | 200 mg/kg/day | 18.0 | 129 |

### Toxicity Experiment

### Method:

Cytarabine Ocfosfate was orally administered to female nude mice in a dose of 53.9 mg/kg/day (total dose of 1131.9 mg/kg) for consecutive 21 days. All of the five mice were alive even 60 days after.

Next, the pharmacological effects of Cytarabine Ocfosfate which is used as the effective ingredient in the present invention are explained, by referring to clinical example.

### Clinical example

1. The patient (male, 71 year old, tissue stage of phase I, no pretreatment) with primary liver cancer (hepatocellular cancer) received a dose of 200 mg/body/day of Cytarabine Ocfosfate as the effective ingredient for consecutive 2 weeks followed by rest for at least 2 weeks. With this procedure as one cycle, administration was repeated. Evaluation of the effects after the two cycles indicated CR (complete response). No significant side effects were noted.
   The evaluation of the effects was made according to the method described in WHO Handbook for Reporting Results of Cancer Treatment, WHO Offset Publication No. 48, World Health Organization Geneva, 1979. The following case was evaluated as well.
2. The patient (female, 61 years old, colectomy, FT (Ftorafur) and FT suppository in pretreatment) with metastatic liver cancer from colony tumor received Cytarabine Ocfosfate as the effective ingredient for one cycle as in Case 1 above. The total evaluation of the effects was MR (minor response).
   Evaluation of the effects by chemotherapy is set forth as follows by WHO Handbook for Reporting Results of Cancer Treatment, WHO Offset Publication No. 48, World Health Organization Geneva, 1979.

Complete response (CR): Complete disappearance of all measurable lesions and secondary lesions caused by tumor, for at least 4 weeks without any new lesion.

Partial response (PR): Estimated decrease in size of a measurable lesion by 50% or more in two directions, or by 30% or more in one way, for at least 4 weeks without aggravation of a secondary lesion or any new lesion.

Minor response (MR): Estimated decrease in size of a measurable lesion by 50% or more in two directions, or by 30% or more in one way, for less than 4 weeks, or decrease in size of a measurable lesion by 25% or more and less than 50% in two directions for at least 4 weeks.

No change (NC): Estimated decrease in size of a measurable lesion by less than 50% in two directions, or by less than 30% or estimated increase within 25% in one way for at least 4 weeks, without aggravation of a secondary lesion caused by tumor and without any new lesion.

Progressive disease (PD): Estimated increase of 25% or more in a measurable lesion or appearance of any new lesion.

Next, the present invention is described in detail with reference to the examples but is not deemed to be limited thereto.

### Example 1 Pharmaceutical preparation

One embodiment of oral composition is shown below but the present invention is not limited thereto. After 103 mg of Cytarabine Ocfosfate was mixed with 157 mg of low substitution hydroxypropyl cellulose as a disintegrator, 3 mg of hydroxypropyl cellulose as a binder, 1 mg of anhydrous sodium carbonate as a stabilizer and 1 mg of magnesium stearate as a lubricant, the mixture was filled up in hard capsule to obtain the capsule preparation for oral administration.

### INDUSTRIAL APPLICABILITY

As described above in detail, Cytarabine Ocfosfate used as the effective ingredient in the present invention exhibits the effect of preventing liver metastasis of cancer and clearly shows an excellent effect for the treatment of primary or metastatic liver cancer. Cytarabine Ocfosfate is thus expected as an agent for preventing liver metastasis of cancer or as a therapeutic agent of liver cancer.

## Claims

1. A pharmaceutical composition for preventing liver metastasis of cancer and pharmaceutical composition for the treatment of liver cancer, comprising as an effective ingredient of Cytarabine Ocfosfate represented by formula (I):

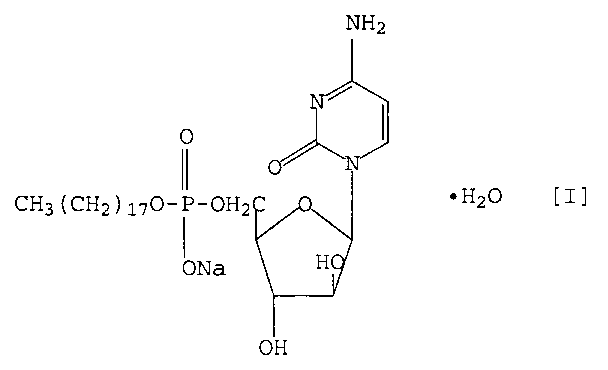

2. A method for preventing liver metastasis of cancer and method for the treatment of liver cancer which comprises administering an effective dose of Cytarabine Ocfosfate represented by formula (I).

3. Use of Cytarabine Ocfosfate for preparing a pharmaceutical composition for preventing liver metastasis of cancer and pharmaceutical composition for the treatment of liver cancer, comprising as an effective ingredient of Cytarabine Ocfosfate represented by formula (I).

4. A process for preparing a pharmaceutical composition for preventing liver metastasis of cancer and pharmaceutical composition for the treatment of liver cancer which comprises mixing Cytarabine Ocfosfate as an effective ingredient with an excipient or a carrier.
